# FASCICULE DE BREVET EUROPEEN

(11) **EP 0 967 947 B1**
(45) Date de publication et mention de la délivrance du brevet: **01.09.2004**
(21) Numéro de dépôt: 98909543.5
(22) Date de dépôt: 16.02.1998
(51) Int. Cl.: A61F 9/007

(54) **IMPLANT DE SCLERECTOMIE PROFONDE**
IMPLANTAT FÜR TIEFE SKLEREKTOMIE
IMPLANT FOR DEEP SCLERECTOMY

(30) Priorité: 17.02.1997 FR 9701801
(43) Date de publication de la demande: 05.01.2000
(73) Titulaire: CORNEAL INDUSTRIE, 74370 Pringy (FR)
(72) Inventeur: TELANDRO, Alain, F-06400 Cannes (FR); SOURDILLE, Philippe, F-44500 La Baule (FR); JALLET, Valérie, F-74960 Meythet (FR); BOS, Gilles, F-74330 La Balme de Sillingy (FR); VILLAIN, Franck, F-74000 Annecy (FR)
(74) Mandataire: Le Roux, Martine
(86) Numéro de dépôt international: PCT/FR1998/000291
(87) Numéro de publication internationale: WO 1998/035639

(56) Documents cités:
- WO-A-89/07426
- WO-A-94/13234
- WO-A-95/35078
- WO-A-96/40005
- GB-A- 2 296 663
- SU-A- 1 066 591
- US-A- 4 716 154
- US-A- 4 946 436
- US-A- 5 433 701
- US-A- 5 558 630

## Description

La présente invention a pour objet un implant de sclérectomie profonde. Ledit implant de l'invention se caractérise par le matériau le constituant et par sa forme.

La technique chirurgicale de sclérectomie profonde a été récemment proposée pour traiter le glaucome. En effet, lorsque le glaucome ne peut être traité médicalement, il est nécessaire d'intervenir chirurgicalement pour réduire la pression intraoculaire.

La plus courante des techniques opératoires pour le traitement du glaucome est la trabéculectomie. Ladite trabéculectomie consiste à pratiquer une ouverture à travers le trabéculum, pour évacuer l'humeur aqueuse de la chambre antérieure vers l'espace sous-conjonctival. Il apparaît alors généralement sous la conjonctive une bulle de filtration provoquée par l'accumulation de ladite humeur aqueuse sous ladite conjonctive . Les complications associées à cette technique chirurgicale sont assez nombreuses. Elles consistent en des hypotonies précoces ou tardives, en des diminutions de profondeur de la chambre antérieure voire en l'effacement de celle-ci, en un soulèvement choroïdien ... Une des complications les plus dramatiques est la rupture secondaire de ladite bulle de filtration, qui peut entraîner une endophtalmie.

D'autres techniques chirurgicales ont été introduites, notamment pour éviter l'ouverture de la chambre antérieure. Une de ces techniques, dans le cadre de laquelle s'inscrit la présente invention, est la sclérectomie profonde dite chirurgie non perforante du glaucome. Elle consiste à inciser la sclère jusqu'à l'aplomb du canal de Schlemm. L'humeur aqueuse sourd alors au travers du trabéculum. Pour aider l'écoulement de ladite humeur aqueuse, il a été proposé de mettre en place, dans ladite incision (dans le lit scléral profond) un implant de collagène, de forme cylindrique (l'extrémité antérieure dudit implant, en regard du canal de Schlemm). Ledit implant facilite ledit écoulement de ladite humeur aqueuse, en jouant le rôle d'une mèche (il transporte les fluides oculaires par capillarité). Des résultats de mise en oeuvre de cette technique (sclérectomie profonde associée à la pose d'un implant de collagène), dont il est rappelé le principe, sont présentés dans Ophtalmologie, 1995 ; 9 : 666-670. Ledit implant, de par sa forme et la nature du matériau le constituant, ne semble pas promis à un grand avenir. En effet, la forme cylindrique n'est pas la forme la plus adaptée à l'anatomie de l'oeil et le matériau utilisé - collagène d'origine animale - n'est plus préconisé au vu des problèmes liés à la maladie de Creutzfelt-Jacob.

Le document WO-A-96/40005 décrit des implants cornéens, destinés-à-être-insérés dans une délamination des tissus. Lesdits implants peuvent être réalisés en de nombreux matériaux, notamment en de l'acide hyaluronique réticulé.

La Demanderesse propose présentement un implant qui, dans le cadre de cette technique chirurgicale de sclérectomie profonde (non perforante) avec implant, se substitue avantageusement audit implant de collagène, de forme cylindrique de l'art antérieur. Ledit implant de l'invention - grâce aux caractéristiques du matériau le constituant et à sa forme géométrique - occupe durablement l'espace créé chirurgicalement et permet efficacement l'écoulement de l'humeur aqueuse hors de la chambre antérieure (sans ouverture de celle-ci) : il constitue un véritable drain.

Ledit implant de l'invention - implant de sclérectomie profonde, destiné à drainer l'humeur aqueuse - est tel que défini dans la revendication 1. Avantageusement, il présente sensiblement la forme d'un polyèdre à cinq ou six faces.

Un tel implant polyédrique qui présente donc au moins cinq sommets se trouve relativement bloqué une fois positionné dans l'espace créé; ledit implant présentant, par ailleurs, bien évidemment un volume adapté au volume dudit espace. Il convient de pouvoir l'insérer dans ledit espace et de l'y voir alors exercer sa fonction de mèche, de véritable drain.

L'homme du métier comprendra parfaitement le qualificatif "sensiblement" employé en référence à la forme des implants de l'invention, au vu de la nature du matériau les constituant. On revient plus loin dans le présent texte sur ledit matériau.

Lesdits implants de l'invention présentent avantageusement sensiblement la forme d'un polyèdre, à au moins cinq faces, convexe ; ledit polyèdre convexe ayant au moins une base sensiblement plane (qui constitue sa face ou l'une de ses faces de plus grande surface) qui présente au moins 3 côtés et une faible épaisseur. On entend par épaisseur desdits implants de l'invention la distance maximale entre ladite base et le sommet opposé ou la face opposée à ladite base. Ladite épaisseur - faible, généralement comprise entre 0,2 et 2 mm - se trouve avantageusement comprise entre 1/12^{ème} et 1/4 (de préférence entre 1/10^{ème} et 1/8^{ème}) de la longueur du plus grand côté de ladite base (à au moins 3 côtés).

Ladite base (sensiblement plane), délimitée par un polygone à au moins 3 côtés, consiste avantageusement en un triangle ou un quadrilatère, plus particulièrement un trapèze (de préférence isocèle).

Ladite base, "sensiblement" plane (de par la nature du matériau constituant l'implant), est toutefois avantageusement légèrement incurvée de sorte que, une fois l'implant mis en place, elle épouse le rayon de courbure de l'oeil. Sa surface est donc, selon cette variante avantageuse, légèrement concave.

Dans le cadre de variantes avantageuses de l'invention, les implants présentent sensiblement :
- la forme d'un prisme, avantageusement droit, à base trapézoïdale (le trapèze de la base d'un tel prisme est de préférence un trapèze isocèle) ou triangulaire;
   ou
- la forme d'un parallélépipède, avantageusement d'un parallélépipède droit, et de préférence d'un parallélépipède rectangle.

Il est clair que, selon d'autres variantes, lesdits implants polyédriques, peuvent présenter d'autres formes et notamment celles de pyramides à base rectangulaire, carrée ou trapézoïdale, celles de telles pyramides tronquées, celles de prismes, notamment droits, à base quelconque ...

On rappelle ici que, de manière générale, la base de l'implant de l'invention (au moins l'une de celles-ci; destinée à être positionnée le long de la membrane de Descemet et de la paroi interne de la sclère) est avantageusement légèrement incurvée.

Par ailleurs, de manière générale également, on a avantageusement les angles du polyèdre constituant l'implant de l'invention, émoussés. Ceci peut constituer un plus en référence à d' éventuels problèmes de traumatismes.

On en vient maintenant à la nature du matériau constituant les implants de l'invention. Il s'agit d'acide hyaluronique réticulé, suffisamment réticulé pour constituer un implant solide.

L'acide hyaluronique est un glycosaminoglycanne ou mucopolysaccharide de poids moléculaire élevé que l'on trouve dans les tissus animaux tels que les cordons ombilicaux, l'humeur vitrée, le liquide synovial, les crêtes de coq, la peau, les tissus connectifs (articulations , tendons ...) ... Ledit acide peut ainsi être obtenu naturellement par extraction à partir de certains desdits tissus animaux (des crêtes de coq et cordons ombilicaux notamment). Il peut également être obtenu par fermentation bactérienne. Ledit acide possède une grande propension à absorber l'eau. Il s'agit d'un matériau très hydrophile dont la teneur en eau, à l'équilibre, est supérieure à 99 %. Ledit matériau convient parfaitement pour assurer la fonction de drain recherchée pour l'implant de l'invention.

La structure chimique dudit acide est celle d'un polymère présentant des monomères disaccharidiques de N-acétyl-D-glucosamine et d'acide-D-glucoronique, ladite amine et ledit acide étant reliés par une liaison glucosidique β1 → 3. Les monomères disaccharidiques sont eux reliés entre eux par des liaisons glucosidiques β1 → 4 pour générer la chaîne polysaccharidique non réticulée, sans embranchement.

Ladite chaîne présente toutefois, au niveau de ses monomères, des fonctions, notamment hydroxyles, qui permettent de la réticuler chimiquement afin de créer un réseau plus ou moins dense.

Dans le présent texte et les revendications qui y sont annexées, on emploie le terme acide hyaluronique comme nom générique pour désigner aussi bien l'acide hyaluronique per se que ses sels et notamment les sels de hyaluronate. Les implants de l'invention sont donc à base d'un polymère choisi parmi l'acide hyaluronique réticulé et les sels réticulés dudit acide. Avantageusement, ils sont à base de hyaluronate de sodium réticulé. Ledit hyaluronate de sodium intervenant est avantageusement d'origine bactérienne.

Ledit acide hyaluronique intervient, dans le cadre de l'invention, solide, réticulé à un taux de réticulation suffisant. On préconise de mettre en oeuvre ladite réticulation, via les fonctions hydroxyles dudit acide, au moyen d'un agent réticulant présentant des fonctions réactives; ledit agent intervenant en des quantités telles que le rapport : nombre total de fonctions réactives dudit agent réticulant (intervenant dans le milieu réactionnel) / nombre total de motifs disaccharidiques des molécules d'acide hyaluronique (présentes dans le milieu réactionnel) soit compris entre 0,2 et 1.

Une réticulation minimale assure l'effet escompté : l'obtention d'un solide, présentant un minimum de résistance mécanique.

Une réticulation maximale n'est pas, avantageusement, dépassée. L'intervention d'une quantité importante d'agent réticulant dénature en effet les implants de l'invention.

A titre d'agent réticulant, on peut faire intervenir, pour générer les implants de l'invention, tout agent connu pour réticuler l'acide hyaluronique par l'intermédiaire de ses fonctions hydroxyles - agent réticulant au moins bifonctionnel - et notamment un polyépoxyde ou ses dérivés. A titre de tel agent réticulant, on peut notamment faire intervenir l'épichlorhydrine, le divinylsulfone, le 1,4-bis(2,3-époxypropoxy)butane (ou 1,4-bis(glycidyloxy)butane ou encore 1,4-butanediol diglycidyl éther = BDDE), le 1,2-bis(2,3-époxypropoxy)éthylène, le 1-(2,3-époxypropyl)-2,3-époxy cyclohexane ... De nombreux agents réticulants convenant pour la mise en oeuvre de la réticulation de l'acide hyaluronique ont notamment été décrits dans le brevet US-A-4,716,154.

Il n'est pas exclu du cadre de l'invention de faire intervenir plusieurs agents réticulants pour obtenir un acide hyaluronique réticulé dans lequel on taille un implant de l'invention.

Selon une variante avantageuse, on utilise pour la préparation dudit acide hyaluronique réticulé un agent de réticulation dont les fonctions réactives sont des fonctions époxy.

D'une manière générale, la mise en oeuvre de la réticulation de l'acide hyaluronique (ou d'un de ses sels) est un procédé familier à l'homme du métier.

De façon originale, dans le cadre de la présente invention, on utilise ledit acide hyaluronique réticulé pour générer des implants polyédriques à au moins cinq faces, convenant à titre d'implants de sclérectomie profonde. Lesdits implants, de par le matériau les constituant, sont très hydrophiles et assurent efficacement l'évacuation de l'humeur aqueuse hors de la chambre antérieure.

Les implants de l'invention - polyèdres à au moins 5 faces, en acide hyaluronique réticulé - sont éventuellement chargés en au moins un principe actif. Ledit principe actif peut notamment consister en un antibiotique et/ou un antimitotique. Le chargement en principe(s) actif(s) de la masse desdits implants de l'invention ne soulève aucune difficulté particulière. Il est généralement mis en oeuvre au cours d'une étape d'hydratation desdits implants.

Enfin, les implants de l'invention, sont obtenus par découpe de la forme adéquate dans un bloc d'acide hyaluronique réticulé. Ledit bloc, présentant généralement une faible épaisseur, on parle plutôt de lamelle. Les implants de l'invention sont mis en place sans difficulté particulière au travers d'une incision adéquate. Ladite incision a été préalablement pratiquée pour réaliser l'espace ou cavité d'implantation.

La présente invention couvre également une application originale - à titre d'implant de sclérectomie profonde, destiné à drainer l'humeur aqueuse - d'une masse sensiblement polyédrique à au moins cinq faces (avantageusement à cinq ou six faces), en acide hyaluronique réticulé.

Elle a, en fait, également pour objet :
- une utilisation originale, de l'acide hyaluronique réticulé, pour la production d'un implant de sclérectomie profonde, présentant sensiblement la forme d'un polyèdre à au moins cinq faces, avantageusement sensiblement la forme d'un polyèdre à cinq ou six faces ; implant destiné à drainer l'humeur aqueuse ;
- la mise en oeuvre de la sclérectomie profonde - chirurgie non perforante du glaucome - avec intervention d'un implant, en le matériau précité, de la forme précitée.

Pour illustrer, de façon nullement limitative, l'invention présentement revendiquée, on annexe à la description ci-dessus un exemple de préparation d'implants de l'invention.

Le protocole suivi est le suivant :
- 1,00 g de hyaluronate de sodium (d'une masse moléculaire de 2.106 Da) est dissous dans 7,80 g d'une solution aqueuse de soude à 0,25 M ;
- 0,192 g de BDDE (réticulant : 1,4-butanediol diglycidyl éther) sont ajoutés à la solution ;
- après homogénéisation, celle-ci est mise au bain-marie à 50°C pendant 2 heures ;
- un gel solide est alors obtenu qui est hydraté jusqu'à équilibre dans de l'eau désionisée;
- ledit gel est alors purifié par extraction en continu, par de l'eau désionisée, dans un soxhlet ;
- le gel ainsi purifié est mis en équilibre dans une solution de tampon phosphate à pH 7,2 ;
- on découpe alors dans celui-ci des lamelles de 0,5 à 1 mm d'épaisseur ;
- on trépane enfin ces lamelles , à l'aide d'un trépan présentant une section trapézoïdale (trapèze isocèle dont les bases mesurent respectivement 3 et 0,5 mm et la hauteur 5 mm) : on obtient alors des implants qui ont sensiblement la forme de prismes droits à base trapézoïdale et dont l'épaisseur varie entre 0,5 et 1 mm.

## Revendications

1. Implant de sclérectomie, profonde, destiné à drainer l'humeur aqueuse, en acide hyaluronique réticulé et présentant sensiblement la forme d'un polyèdre à au moins cinq faces ; ledit acide hyaluronique ayant été réticulé, via ses fonctions hydroxyles, au moyen d'un agent réticulant présentant des fonctions réactives ; ledit agent étant intervenu en des quantités telles que le rapport : nombre total de fonctions réactives dudit agent réticulant/nombre total de motifs disaccharidiques des molécules d'acide hyaluronique est compris entre 0,2 et 1.

2. Implant selon la revendication 1, **caractérisé en ce qu'**il présente sensiblement la forme d'un polyèdre à cinq ou six faces.

3. Implant selon l'une des revendications 1 ou 2, **caractérisé en ce qu'**il présente sensiblement la forme d'un polyèdre convexe ; ledit polyèdre convexe ayant au moins une base sensiblement plane, qui présente au moins 3 côtés, et une épaisseur - distance maximale entre ladite base et le sommet opposé à ou la face opposée à ladite base - faible.

4. Implant selon la revendication 3, **caractérisé en ce que** ladite épaisseur dudit polyèdre convexe est comprise entre 1/12^{ème} et ¼ de la longueur du plus grand côté de ladite base.

5. Implant selon l'une des revendications 3 ou 4, **caractérisé en ce que** ladite base a une forme triangulaire ou trapézoïdale.

6. Implant selon l'une quelconque des revendications 3 à 5, **caractérisé en ce que** ladite base est légèrement incurvée de sorte que, une fois l'implant mis en place, elle épouse le rayon de courbure de l'oeil.

7. Implant selon l'une quelconque des revendications 1 à 6, **caractérisé en ce qu'**il présente sensiblement la forme d'un prisme.

8. Implant selon la revendication 7, **caractérisé en ce qu'**il présente sensiblement la forme d'un prisme droit, à base trapézoïdale.

9. Implant selon l'une quelconque des revendications 1 à 8, **caractérisé en ce que** ses sommets sont émoussés.

10. Implant selon l'une quelconque des revendications 1 à 9, **caractérisé en ce que** lesdites fonctions réactives dudit agent réticulant sont des fonctions époxy.

11. Implant selon l'une quelconque des revendications 1 à 10, **caractérisé en ce que** ledit acide hyaluronique réticulé est chargé en au moins un principe actif.

## Patentansprüche

1. Tiefes (nicht perforierendes) Sklerektomie-Implantat, das zur Drainage des Augenwassers bestimmt ist und besteht aus vernetzter Hyaluronsäure und im Wesentlichen die Form eines Polyeders mit mindestens 5 Flächen aufweist, wobei die Hyaluronsäure über ihre Hydroxyl-Funktionen mit einem Vernetzungsmittel, das reaktionsfähige Funktionen aufweist, vernetzt worden ist, wobei dieses Mittel in solchen Mengen verwendet worden ist, dass das Verhältnis zwischen der Gesamtanzahl der reaktionsfähigen Funktionen des Vernetzungsmittels und der Gesamtanzahl der wiederkehrenden Disaccharideinheiten der Hyaluronsäure-Moleküle zwischen 0,2 und 1 liegt.

2. Implantat nach Anspruch 1, **dadurch gekennzeichnet, dass** es im Wesentlichen die Form eines Polyeders mit 5 oder 6 Flächen aufweist.

3. Implantat nach einem der Ansprüche 1 oder 2, **dadurch gekennzeichnet, dass** es im Wesentlichen die Form eines konvexen Polyeders aufweist, der mindestens eine im Wesentlichen ebene Basis hat, die mindestens drei Seiten aufweist und eine geringe Dicke - entsprechend dem maximalen Abstand zwischen der Basis und der Spitze, die der Basis gegenüberliegt, oder der Fläche, die der Basis gegenüberliegt - hat.

4. Implantat nach Anspruch 3, **dadurch gekennzeichnet, dass** die Dicke des konvexen Polyeders zwischen 1/12 und 1/4 der Länge der größten Seite der Basis liegt.

5. Implantat nach einem der Ansprüche 3 oder 4, **dadurch gekennzeichnet, dass** die Basis eine dreieckige oder trapezförmige Form hat.

6. Implantat nach einem der Ansprüche 3 bis 5, **dadurch gekennzeichnet, dass** die Basis leicht nach innen gekrümmt ist in der Weise, dass sich das Implantat, wenn es einmal eingesetzt ist, sich an den Krümmungsradius des Auges anschmiegt.

7. Implantat nach einem der Ansprüche 1 bis 6, **dadurch gekennzeichnet, dass** es im Wesentlichen die Form eines Prismas hat.

8. Implantat nach Anspruch 7, **dadurch gekennzeichnet, dass** es im Wesentlichen die Form eines geraden Prismas mit trapezförmiger Basis hat.

9. Implantat nach einem der Ansprüche 1 bis 8, **dadurch gekennzeichnet, dass** seine Spitzen stumpf bzw. abgestumpft sind.

10. Implantat nach einem der Ansprüche 1 bis 9, **dadurch gekennzeichnet, dass** die reaktionsfähigen Funktionen des Vernetzungsmittels Epoxy-Funktionen sind.

11. Implantat nach einem der Ansprüche 1 bis 10, **dadurch gekennzeichnet, dass** die vernetzte Hyaluronsäure mit mindestens einem Wirkstoff beladen ist.

## Claims

1. Implant for deep sclerectomy, intended to drain the aqueous humour, made of crosslinked hyaluronic acid and presenting substantially the shape of a polyhedron with at least five faces, said hyaluronic acid having been crosslinked, via its hydroxyl functions, by means of a crosslinking agent, presenting reactive functions, used in quantities such that the ratio: total number of reactive functions of said crosslinking agent/total number of disaccharidic repeating units of the molecules of hyaluronic acid is included between 0.2 and 1.

2. The implant according to claim 1, **characterized in that** it presents substantially the shape of a polyhedron with five or six faces.

3. The implant according to claim 1 or 2, **characterized in that** it presents substantially the shape of a convex polyhedron; said convex polyhedron having at least a substantially plane base which presents at least 3 sides and a small thickness; said thickness being defined as the maximum distance between said base and the apex opposite or the face opposite said base.

4. The implant according to claim 3, **characterized in that** said thickness of said convex polyhedron is included between 1/12^{th} and 1/4 of the length of the largest side of said base.

5. The implant according to claim 3 or 4, **characterized in that** said base has a triangular or trapezoidal shape.

6. The implant according to any one of claims 3 to 5, **characterized in that** said base is slightly incurved so that, once the implant is positioned, it follows the radius of curvature of the eye.

7. The implant according to any one of claims 1 to 6, **characterized in that** it presents substantially the shape of a prism.

8. The implant of claim 7, **characterized in that** it presents substantially the shape of a straight prism with trapezoidal base.

9. The implant according to any one of claims 1 to 8, **characterized in that** its apices are blunt.

10. The implant according to any one of claims 1 to 9, **characterized in that** said reactive functions of said crosslinking agent are epoxy functions.

11. The implant according to any one of claims 1 to 10, **characterized in that** said crosslinked hyaluronic acid has at least one active principle incorporated therein.
